# EUROPEAN PATENT SPECIFICATION

(11) **EP 1 325 503 B1**
(45) Date of publication and mention of the grant of the patent: **31.10.2007**
(21) Application number: 01967515.6
(22) Date of filing: 19.09.2001
(51) Int. Cl.: G21K 1/04

(54) **RADIOTHERAPY APPARATUS AND COLLIMATOR SET THEREFOR**
APPARAT ZUR RADIOTHERAPIE UND ZUGEHÖRIGE KOLLIMATORANORDNUNG
APPAREIL DE RADIOTHERAPIE ET ENSEMBLE COLLIMATEUR DESTINE A CET APPAREIL

(30) Priority: 11.10.2000 GB 0024843
(43) Date of publication of application: 09.07.2003
(73) Proprietor: Elekta AB (publ), 103 93 Stockholm (SE)
(72) Inventor: BROWN, Kevin, John, West Sussex RH13 5EJ (GB); STREAMER, Ralph, Peter, Horsham, West Sussex RH12 5RS (GB)
(74) Representative: Downing, Michael Philip
(86) International application number: PCT/GB2001/004176
(87) International publication number: WO 2002/031837

(56) References cited:
- EP-A- 0 314 214
- US-A- 3 969 629
- US-A- 4 672 212
- US-A- 5 165 106
- US-A- 5 166 531
- LIND B ET AL: "DEVELOPMENT OF TREATMENT TECHNIQUES FOR RADIOTHERAPY OPTIMIZATION" INTERNATIONAL JOURNAL OF IMAGING SYSTEMS AND TECHNOLOGY, WILEY AND SONS, NEW YORK, US, vol. 6, no. 1, 1 March 1995 (1995-03-01), pages 33-42, XP000620332 ISSN: 0899-9457

## Description

### FIELD OF THE INVENTION

The present invention relates to a radiotherapy apparatus, in particular to the arrangement of collimators within the radiation head.

### BACKGROUND ART

In a conventional multi-leaf collimator (MLC), the radiation beam is collimated by an array of thin leaves lying alongside each other which can each be extended longitudinally to define a unique edge. The leaves move in a given direction (Y) and generally there are two sets of additional backup diaphragms orthogonal to this (X). These are solid and move in and out in the X and Y directions. They perform two functions. The X diaphragm allows the field edge to be adjusted in a continuous manner, whereas the leaves alone would only allow discrete adjustments a leaf width at a time. The Y diaphragm reduces the effect of leakage through the leaves. The X diaphragm also shields the gap between leaves that are out of the treatment field and are effectively 'closed'.

This arrangement is shown in figure 1, a head and collimator set of the type shown in EP-A-0 314 214. An X-ray source 10 is placed behind a primary collimator 12 which allows through a divergent cone of radiation 14. The beam is modified by a combination of a filter, an ion chamber and a wedge 16 before passing through a mirror 18 placed at an angle to the beam axis. This provides a view down the beam for a camera located at 20. Thus, the camera is able to see the position of the subsequent collimators for checking purposes.

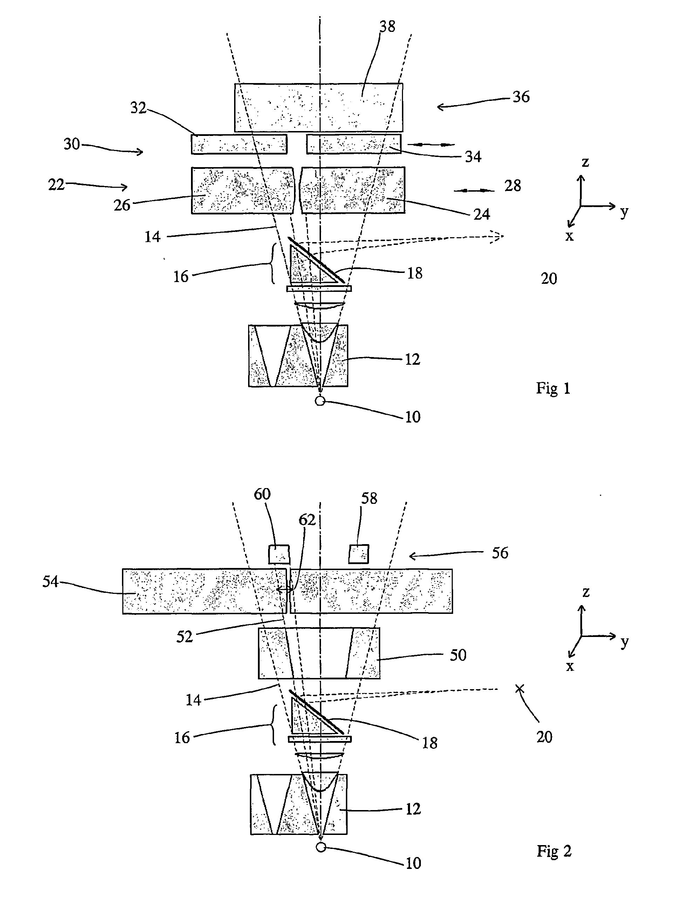

After the mirror 18, the beam is modulated by a multi-leaf collimator (MLC) 22 formed of arrays of opposing leaves 24,26. These can move longitudinally in a Y direction from left to right in figure 1 as shown by arrows 28. A large number of narrow leaves forms an array stretching into and out of the figure. Thus, by moving each leaf to a desired position, the array forms a collective edge which collimates the beam.

Subsequent to the MLC 22 is a Y collimator. This consists of a pair of jaws 32, 34 which each extend across the width of the multi-leaf array 22 and can be moved in and out in the same Y direction as the leaves 24, 26 of the MLC array 22. These leaves therefore lie behind the leaves of the MLC 22 and limit leakage of radiation between the individual leaves.

Finally, an X collimator 36 comprises a pair of jaws similar to those of the Y collimator but deeper and displaced by 90°. One such jaw 38 is visible in figure 1. The jaws of the X collimators move transversely to the leaves of the MLC 22. To avoid collisions between opposing leaves in the MLC array 22, a minimum approach distance is defined which provides a minimum gap between leaves in the Y direction. This however creates a gap in the collimation. To cover this, the leaves are positioned so that this gap lies remote from the treatment area, and the relevant X collimator jaw is advanced to cover it. The X collimator can also trim the radiation field in the X direction by fractional leaf widths. The jaws are deeper since they must in places provide full attenuation of the beam, as opposed to the Y collimator which provides a secondary attenuation after the MLC 22.

These moving diaphragms together with their corresponding bearings and readout systems introduce significant complexity into the design and extend the depth of the apparatus, reducing the clearance between it and the patient. The present invention seeks to address these issues.

Besides EP-A-0314214, mentioned above, the prior art discloses other collimator arrangements.

US-A-4,672,212 discloses a radiation head having a primary collimator 9 and a multi-leaf collimator, together with block collimators 7 between the primary collimator 9 and the multi-leaf collimator. The block collimators 7 operate in the direction transverse to the direction of movement of the multi-leaf collimator.

US-A-5,166,531 discloses a radiation head with a multi-leaf collimator.

US-A-5,165,106 discloses a multi-leaf collimator in which individual leaves are rotated around a journal 28 in order to bring them into and out of the radiation beam.

"Development of Treatment Techniques for Radiotherapy Optimisation" by Lind and Brahme, International Journal of Imagery Systems and Technology, Volume 6, 33-42 (1995) discusses various forms of non-uniform dose delivery including dynamic multi-leaf collimation, and discloses a radiation head including a primary collimator, block collimators, and multi-leaf collimators acting in a direction transverse to the block collimators.

US-A-3,969,629 discloses a radiation head having a primary collimator 15, a secondary collimator 18, and a pair of transversely arranged block collimators 19, 20.

### SUMMARY OF THE INVENTION

The present invention therefore provides a radiation head as set out in claim 1.

This means that to close a pair of opposing leaves, they are moved to their minimum separation, with the gap being covered by the leaf edge collimator. Thus the former X collimator is unnecessary.

In this arrangement, the MLC is after the aperture collimator. In combination with thin leaves, the MLC leaves will project a much reduced leaf width at the Isocentre of the radiotherapy apparatus. As a result, collimation of the radiation field by fractional leaf widths becomes unnecessary. Such leaves can also be focussed at a point slightly offset relative to the target, thereby reducing leakage between leaves to a clinically acceptable level and removing the need for the former Y collimator.

The leaf edge collimator is preferably fixed. This will mean that the complexity of a moveable collimator with its associated bearings, drive mechanism etc will have been removed. The aperture collimator can act in the X direction also, and in practice this will usually be the case.

The aperture collimator could be integrated into the primary collimators, filters etc, but need not be. In practice it is likely to be more practical to provide a fresh collimator set that can be employed with existing sources. These will often include a primary collimator, filter, camera etc.

The projected difference in the Y direction between the first and second extent is preferably not less than the projected minimum approach of opposing leaves of the MLC array. This ensures adequate cover for the opposing leaf gap by the leaf edge collimator. Preferably the projected difference is more than twice or more than three times the projected minimum approach, to allow for positioning tolerances.

The present invention also relates to a radiotherapy apparatus comprising a radiation head as defined above.

### BRIEF DESCRIPTION OF DRAWINGS

An embodiment of the present invention will now be described by way of example, with reference to the following figures, in which;
Figure 1, already described, shows a vertical section through a conventional radiation head with collimators;
Figure 2 shows a corresponding view of a radiation head according to the present Invention; and
Figure 3 shows a view from beneath of the radiation head of figure 2.

### DETAILED DESCRIPTION OF THE EXAMPLE

Referring to figure 2, the present invention provides a simplified method of using an MLC by replacing the moving X and Y diaphragms with fixed diaphragms before and after the MLC, as shown in Fig 2. It also maintains the conventional configuration of primary collimator, filter and ion chamber.

Thus, a radiation source 10 emits x-rays which are limited to a divergent beam 14 by a primary collimator 12 and adjusted by a filter, ion chamber and wedge 16. A fixed aperture collimator 50 then collimates the beam to an intermediate field size 52 in the X and Y directions.

A multi-leaf collimator 54 is arranged after the aperture collimator 50. The maximum field of the MLC 54 is slightly less than the intermediate field size 52. As a result, the position of all leaves in the MLC array 54 can be seen by a camera at 20 via mirror 18, as in the known arrangement of figure 1. This provides an important re-assurance during operation that the apparatus is operating correctly.

Beyond the MLC 54, a leaf edge collimator 56 comprises two solid fixed bars 58, 60, aligned in the X direction and hence transverse to the leaves of the MLC 54. These are positioned so as to protrude into the intermediate field size 52 from the outside thereof by an amount which projects at the isocentre greater than the projected minimum approach of opposing leaf pairs of the MLC array 54. To provide room for usual positioning tolerances, in the example the bars are three times this minimum size. A ratio of two will often be adequate. Thus, when one leaf is fully withdrawn or nearly so and its opposing leaf is extended fully to the minimum approach, or nearly so, the gap 62 between them will be covered by the leaf edge collimator 56.

In this way, and in the ways set out above, moving X and Y collimators are obviated in favour of simple fixed collimators.

The leaf edge collimator is employed in the embodiment of figure 2 since the optical readout system employed to observe the position of the leaves of the MLC needs to have a clear line of sight to the ends of the leaves. In a system in which alternative readout systems are used, the closed leaves could be positioned above the aperture collimator and the leaf edge collimator would be unnnecessary.

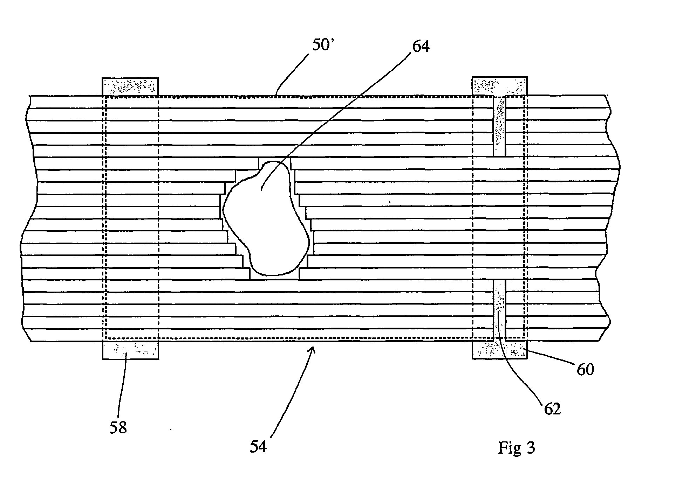

Figure 3 shows for illustration the view of a camera at location 20 with the aperture collimator 50 removed. Dotted lines 50' show the location of the aperture collimator 50. A tumour (for example) is located at 64 and the leaves of the MLC 54 are opened sufficient to allow a clear line of transmission to the tumour. Outside the field of the tumour, the leaves of the MLC 54 are extended or retracted as necessary such that their tips overlie the leaf edge collimator 60. Accordingly the gap 62 between opposing leaf tips is covered by the leaf edge collimator 60. The opposing leaf edge collimator 58 is redundant but offers flexibility in use as full leaf travel can be slow.

It will be appreciated by those skilled in the art that many variations can be made to the above described embodiment without departing from the present invention.

## Claims

1. A radiation head for a radiotherapy apparatus comprising, in sequence, In the direction of the beam, a primary collimator, an aperture collimator (50), a multi-leaf collimator (54) with a pair of opposing arrays of elongate leaves each moveable longitudinally in a Y direction, and a leaf edge collimator (56), the aperture collimator (50) being fixed and adapted to collimate the beam (14) in the Y direction to an extent, and the leaf edge collimator (56) being adapted to further collimate the extent of the beam (14) in the Y direction to a second and therefore lesser extent.

2. A radiation head according to claim 1, in which the leaf edge collimator (56) is fixed.

3. A radiation head according to claim 1 or claim 2 in which the projected difference in the Y direction between the first and second extent is not less than the projected minimum approach of opposing leaves of the MLC array.

4. A radiation head according to claim 3 in which the projected difference is more than twice the projected minimum approach.

5. A radiation head according to claim 3 in which the projected difference is more than three times the projected minimum approach.

6. A radiation head according to any preceding claim in which the aperture collimator (50) serves to collimate the beam (14) in the X direction to an extent.

7. A radiotherapy apparatus comprising a radiation head according to any preceding claim.

## Patentansprüche

1. Strahlerkopf für ein Strahlentherapiegerät, umfassend reihenfolglich, in der Richtung des Strahls, einen Primärkollimator, einen Lochkollimator (50), einen Multileaf-Kollimator (54) mit einem Paar gegenüberliegenden Arrays von Längslamellen, wobei jede longitudinal in einer Y-Richtung beweglich ist, und einen LamellenkantenKollimator (56), wobei der Lochkollimator (50) zum Kollimieren des Strahls (14) in einem gewissen Ausmaß in der Y-Richtung fixiert und angepasst ist, und wobei der Lamellenkantenkollimator (56) angepasst ist, um den Strahl (14) in einem gewissen Ausmaß in der Y-Richtung in einem zweiten und deshalb geringeren Ausmaß weiter zu kollimieren.

2. Strahlerkopf nach Anspruch 1, worin der Lamellenkantenkollimator (56) fixiert ist.

3. Strahlerkopf nach Anspruch 1 oder 2, worin der projizierte Unterschied in der Y-Richtung zwischen dem ersten und zweiten Ausmaß nicht geringer ist als das projizierte minimale Aufeinanderzubewegen der sich gegenüberliegenden Lamellen des MLC-Arrays.

4. Strahlerkopf nach Anspruch 3, worin der projizierte Unterschied mehr als das Zweifache des projizierten minimalen Aufeinanderzubewegens beträgt.

5. Strahlerkopf nach Anspruch 3, worin der projizierte Unterschied mehr als das Dreifache des projizierten minimalen Aufeinanderzubewegens beträgt.

6. Strahlerkopf nach einem der vorangehenden Ansprüche, worin der Lochkollimator (50) in einem gewissen Ausmaß zum Kollimieren des Strahls (14) in der X-Richtung dient.

7. Strahlentherapiegerät, umfassend einen Strahlerkopf nach einem der vorangehenden Ansprüche.

## Revendications

1. Tête d'irradiation pour un appareil de radiothérapie comprenant, dans l'ordre suivant et en allant dans la direction du faisceau, un collimateur primaire, un collimateur à ouverture (50), un collimateur multilames (54) avec une paire de groupes opposés de lames allongées, chacune pouvant être déplacée longitudinalement dans une direction Y, et un collimateur de bords de lames (56), le collimateur à ouverture (50) étant fixe et adapté pour collimater le faisceau (14) jusqu'à une certaine étendue dans la direction Y, et le collimateur de bords de lames (56) étant adapté pour collimater davantage l'étendue du faisceau (14) dans la direction Y jusqu'à une seconde - et donc moindre - étendue.

2. Tête d'irradiation selon la revendication 1, dans laquelle le collimateur de bords de lames (56) est fixe.

3. Tête d'irradiation selon la revendication 1 ou la revendication 2, dans laquelle la différence projetée entre la première et la seconde étendues dans la direction Y n'est pas inférieure à l'approche minimum projetée de lames opposées de l'ensemble collimateur multilames.

4. Tête d'irradiation selon la revendication 3, dans laquelle la différence projetée égale plus du double de l'appproche minimum projetée.

5. Tête d'irradiation selon la revendication 3, dans laquelle la différence projetée égale plus du triple de l'approche minimum projetée.

6. Tête d'irradiation selon l'une quelconque des revendications précédentes, dans laquelle le collimateur à ouverture (50) sert à collimater le faisceau (14) jusqu'à une certaine étendue dans la direction X.

7. Appareil de radiothérapie qui comprend une tête d'irradiation selon l'une quelconque des revendications précédentes.
